# EUROPEAN PATENT APPLICATION

(11) **EP 1 832 649 A1**
(43) Date of publication of application: **12.09.2007**
(21) Application number: 06380278.9
(22) Date of filing: 27.10.2006
(51) Int. Cl.: C12F 3/06, C12F 3/00, C07C 59/255, C07C 51/02

(54) **Method for treating wine-alcohol industry liquid discharges**

(30) Priority: 09.03.2006 ES 200600599
(71) Applicant: Pedros Llevata, Francisco, 46007 Valencia (ES)
(72) Inventor: Pedros Llevata, Francisco, 46007 Valencia (ES)
(74) Representative: Carpintero Lopez, Francisco

(57) **Abstract**

The invention provides a method for treating a liquid discharge from the wine-alcohol industry comprising: a) putting the discharge in contact with an anionic exchange resin and obtaining a cationic solution with potassium cations and chloride anions that is concentrated in order to obtain a potassium-enriched solution that is useful as a fertilizer. It also comprises regenerating the anionic exchange resin with a sodium chloride solution, obtaining an anionic solution of tartrate, from which either a tartaric acid-enriched solution or a calcium tartrate precipitate is obtained.

## Description

### FIELD OF THE INVENTION

The present invention refers to a method for treating liquid discharges originating from the wine-alcohol industry such as vinasse or tank wash water. Said treatment allows discharge elimination and simultaneous recovery of a potassium ion-rich fraction that can be used as a fertilizer and a tartrate ion-rich fraction that can be used in the same wine-alcohol industry as an additive for acidity correction in wines or in manufacturing calcium tartrate.

### BACKGROUND OF THE INVENTION

The wine-alcohol industry exploits wine-making by-products such as wine marcs, lees, and surpluses obtaining alcohols of varying degrees by distillation, grape pips or seeds (of dealcoholized marc) by drying and sieving and calcium tartrate by precipitation and drying. Dry, seed-free marc is burnt, at least partially covering energy needs. Alcohol is obtained exclusively from wine surplus. In some cases electric energy is further obtained by vapor turbines from marc biomass.

Likewise, the wine-alcohol industry generates a great amount of liquid discharges comprising vinasses and winery discharges, including tank and machinery wash waters and discharges from rectified must manufacture.

These discharges usually constitute a serious environmental problem due to their large volume and their chemical characteristics, which make treatment by conventional methods difficult, particularly due to the high content in tartrate anion and potassium cation in solution, both in vinasses and in winery discharges. Tartrate anion is a good masking agent which forms stable complexes with a large amount of divalent, trivalent and tetravalent cations, disturbing systematic cation analytical tests. Tartrate is an anion that is considered to be a reducer since it reduces the permanganate anion in a hot acid medium, by oxidizing itself. Since tartrate is on one hand poorly oxidable (it only decolorizes to permanganate under heat) and since on the other hand it forms very stable complexes with many cations, conventional depuration treatments are not successful.

The usual destination of vinasses has been their spillage as waste to the environment, with the respective uncontrolled pollution this entails. Currently, vinasses are taken to decanting reservoirs for pouring over fields under hydrographic basin control according to liters per square meter per year, there being limits when the discharge is discharged to municipal sewer systems. Alternatively, vinasses are filtered separating insoluble organic and inorganic substances from soluble ones in the physico-chemical conditions of the operation. Thus a wet solid phase is obtained that is eliminated either as field fertilizer or by mixing it with marc for composting, and a brown-colored liquid phase with Chemical Oxygen Demand values between 10.000 and 24.000 mg oxygen/liter and values between 5.000 and 11.000 mg of total organic carbon (TOC)/liter.

Other alternative methods have been developed for eliminating vinasses such as aerobic and anaerobic purification treatments, comprising the use of polyelectrolytes as coagulants and flocculants, which, besides being expensive, are not successful, or evaporation and biogas generation reservoirs.

However, despite the different treatments that exist for vinasses, the generation of large amounts of vinasses and other liquid discharges makes it necessary to develop alternative methods for their treatment that are equally or more effective than those in the state of the art in order to reduce the impact thereof on the environment. It would be especially desirable to have an alternative treatment method for this waste that would be capable of eliminating them on the one hand, at the same time as they revaluate, by recovering products with added value and commercial interest.

In this context, the present inventors have found a method for treating wine-alcohol industry liquid wastes, and especially vinasses, that has the advantage that it allows obtaining a potassium-rich product useful as fertilizer and a tartrate-rich product that is very advantageous for use, amongst other things, in the very field of the wine-alcohol industry, as an additive for wine acidity correction or in manufacturing calcium tartrate.

Said method is also useful for treating other wine-alcohol industry wastes, such as rectified must discharges and recovery, from said wastes, of the aforementioned value-added products.

The method of the present invention achieves eliminating a problematic and polluting dischrage and recovering and revaluating components from wine-alcohol industry liquid discharges.

### OBJECT OF THE INVENTION

An object of the present invention relates to a method for treating liquid discharges from the wine-alcohol industry that allows the simultaneous recovery of value-added solutions such as a potassium ion-rich solution and a tartaric acid-enriched solution or a calcium tartrate precipitate.

Another object of the invention relates to a potassium-enriched solution obtained by the method of the invention.

Another object of the invention relates to a tartaric acid-enriched solution obtained by the method of the invention.

A last object of the invention relates to a calcium tartrate precipitate obtained by the method of the invention.

### BRIEF DESCRIPTION OF THE DRAWINGS

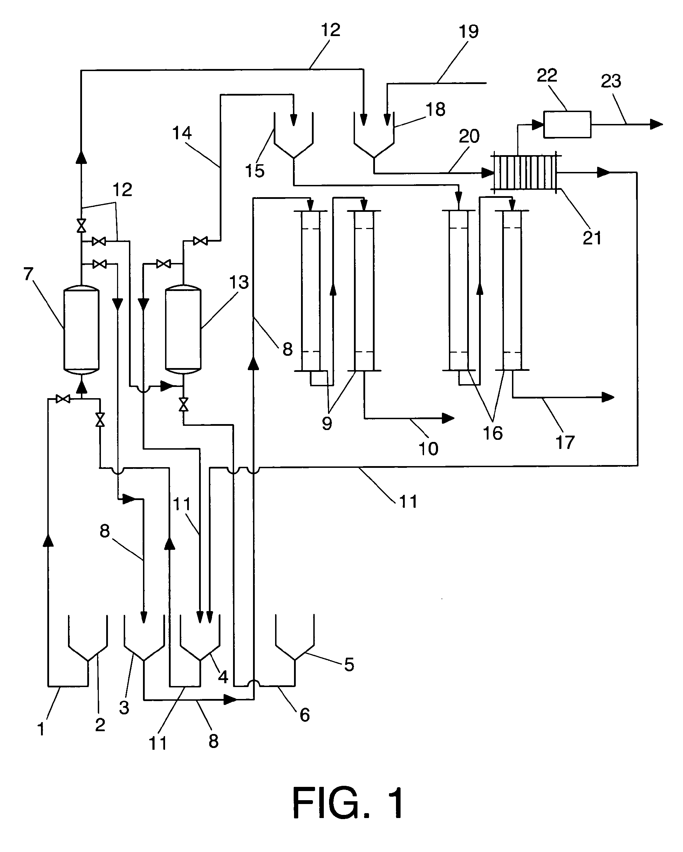

Figure 1 shows an apparatus in which the method of the present invention can take place, in which a liquid discharge (1) is put in contact with an anionic exchange resin (7) in order to obtain a cationic solution (8) comprising potassium cations and chloride anions. The cationic solution (8) is concentrated in an evaporator (9) in order to obtain a potassium-enriched solution (10). The anionic exchange resin (7) is regenerated with a sodium chloride solution (11) and an anionic solution (12) is obtained comprising tartrate anions and sodium cations. This anionic solution (12) can be destined to obtaining a tartaric acid-enriched solution (17) or to obtaining a calcium tartrate precipitate (23). According to the first alternative, the anionic solution (12) is put in contact with a cationic exchange resin (13) in order to obtain a solution (14) comprising tartaric acid, which is then concentrated in an evaporator (16) in order to obtain the tartaric acid-enriched solution (17). According to the second alternative, the anionic solution (12) is put in contact with calcium chloride (19) in order to obtain a calcium tartrate precipitate that is kept in suspension (20), that is filtered in the device (21), dried in the device (22) in order to obtain a calcium tartrate precipitate (23).

### DESCRIPTION OF THE INVENTION

The present invention provides a method, hereinafter method of the invention, for treating liquid discharges from the wine-alcohol industry that allow reducing their environmental impact as well as allowing recovery of the following commercially interesting value-added products: a potassium ion-rich solution for use as a fertilizer, a tartaric acid-enriched solution and/or a calcium tartrate precipitate. The method of the invention comprises the following steps:
a) Putting an anionic exchange resin (7) in contact with said discharge (1) from the wine-alcohol industry in order to obtain a cationic solution (8) comprising potassium cations and chloride anions;
b) Concentrating said cationic solution (8) obtained in the previous step in order to obtain a potassium-enriched solution (10);
c) Regenerating the anionic exchange resin used in step a) with a sodium chloride solution (11); and
d) obtaining an anionic solution (12) comprising tartrate anions and sodium cations.

The method of the invention further comprises obtaining a tartaric acid-enriched solution (17) from the anionic solution (12) obtained in the previous step d) comprising the steps of:
(Ai) putting in contact a cationic exchange resin (13) with the anionic solution (12) in order to obtain a solution (14) comprising tartaric acid; and
(Aii) concentrating said solution (14) in order to obtain the tartaric acid-enriched solution (17).

Alternatively, the method of the invention further comprises obtaining a calcium tartrate precipitate (23) from the anionic solution (12) obtained in previous step d), comprising the steps of:
(Bi) treating the anionic solution (12) with calcium chloride (19) in order to obtain a calcium tartrate precipitate (20) that is kept in suspension; and
(Bii) recovering said precipitate and drying it in order to obtain calcium tartrate (C₄H₄O₆Ca.4H₂0) (23).

The method of the invention can normally start from any liquid waste originating from the wine-alcohol industry, both from the wine making industry and from the vinification by-product exploiting industry, characterized in that it presents tartrate anions and potassium cations. In a preferred embodiment, said discharge is selected from a pomace vinasse, a wine vinasse, a lees vinasse, winery tank-wash water, winery machine wash water, and is subjected to the method of the invention separately, especially since said discharges are generated in a series of steps along the wine year. In a more preferred embodiment, the liquid discharge is a vinasse.

Vinasses are considered wines without alcohol, and according to their origin they are classified in pomace vinasses, wine vinasses, and lees vinasses. A pomace is a wine with between 2 and 4 percentage degrees in alcohol volume. It is a hydroalcholic product originating from pressed marc washing. If these are from red wine vinification the product is directly hydroalcoholic (fermented marcs), if they are from white wine vinification (non-fermented marcs) the product is a glucose and fructose solution that ferments as a low-sugar-rich must. A pomace vinasse is an alcohol-free distillation column bottom product that contains all water-soluble atmospheric pressure boiling point nonvolatile organic and inorganic compounds. It is a brown-colored liquid having, expressed in grams/liter: dry extract by evaporation at 100°C 17.82; organic matter (made up of peptic and albuminoidal derivatives, glycerin and other superior alcohols, tannins, and short chain fatty acids) excluding the tartrate anion 10.66; C₄H₄O₆⁻² (tartrate anion) 2.6; C₄H₄O₅⁻² (malate anion) 0.83; K⁺ 1.19; SO₄⁻² 0.45; Ca⁺² 0.31; Mg⁺² 0.11; Fe⁺² 35.10⁻³ ; PO₄⁻³ 0.23; and others: copper, manganese, zinc between 1 and 3 ppm, pH 3.3/3.4.

Wine vinasses are the alcohol-free distillation bottom products, originating from wine distillation destined to obtaining alcohol form wine surpluses (wine for burning). Its composition is very similar to that of pomace vinasse, with lower calcium, magnesium and sulfate levels. Both pomace vinasses and wine vinasses are solutions close to saturation in potassium bitartrate. They normally have a maximum content of 0.0265 M and a minimum content of 0.01 M and a constant and concentration-independent pH of around 3.35.

Lees vinasses. Wine lees or sediment are the precipitate taking place when sugar transforms into alcohol. It is a slurry sediment making up 10% of fermented must volume. Its composition is qualitatively equal to that of the wine it comes from and is industrially characterized not only by alcohol but also by its high tartaric purity, which is the only one currently exploited in calcium tartrate manufacture from lees. This salt is the raw material for producing tartaric acid. Lees contain between 35 and 60 grams/liter of precipitated potassium bitartrate.

They are diluted to between two and three times their volume so that they circulate fluidly enough in the distillation column and the precipitated potassium bitartrate passes to a soluble state at a distillation temperature of approximately 100°C. Lee vinasses abandon the distillation column with a concentration of 20 gr/L or potassium bitartrate and are slowly treated, with stirring, with sulphuric acid and calcium carbonate in order to form calcium tartrate, which is isolated by separation with hydrocyclones (density difference between organic mass and salt), centrifugation (water elimination) and thermal drying. Hydrocyclon effluent and centrifuge water are detartrated lees vinasses. In order to apply the method of the present invention, the acid added must be hydrochloric or preferably only calcium chloride instead of sulphuric acid and calcium carbonate. These detartrated lees vinasses are also called calcium tartrate mother liquors, and they are saturated solutions of 0.01 M calcium tartrate and pH of about 5, containing potassium in the form of 0.1 M potassium chloride. These detartrated lees vinasses or mother liquors, called lees vinasses in the present specification, make up the starting material of the method of the invention.

Lees normally arrive at the alcohol factory in slurry form. There is a not so generalized process in which lees arrive as cakes from filter presses, in which case they are diluted with a large amount of evaporated water and the aforementioned method is followed.

Tank and machinery wash waters have tartrate and potassium and are starting materials for the method of the invention. Tank wash waters, especially, have a usual 1 M concentration of potassium tartrate (C₄H₄O₆K₂). They are alkaline solutions prepared from a 1 M solution of KOH for dissolving the so-called "cream of tartar" consisting of potassium bitartrate crusts that deposit on the metal surfaces of the tanks. They make up a liquid discharge in which potassium added as potassium hydroxide enriches even further the potassium-enriched solution (10) obtained that is useful as soluble liquid fertilizer. The "cream of tartar" is usually supplied to tartaric acid factories as a solid, working at the tanks by hand and/or dissolving it with sodium hydroxide. According to this method a potassium tartrate solution is formed called Seignette salt, the exploiting of which is not organized and is largely spilled in a non-controlled manner. According to the method of the invention, the "cream of tartar" is transformed either into a tartaric acid-enriched solution or into a calcium tartrate precipitate, and the potassium originating from KOH dissolution serves for enriching the soluble liquid fertilizer potassium-enriched solution (10) obtained.

The liquid discharge (1), starting material of the method of the invention is initially filtered in order not to obstruct the exchange resins. Laboratory filter paper for qualitative analysis with a pore size comprised between 20-25 microns is usually suitable for filtration. Suitable industrial equipment are press filters with membrane plates and fabric of suitable porosity. In a preferred embodiment, press filters with membrane plates are used since they shorten the filtration cycle and cake compaction is carried out by means of membrane swelling by means of a hydraulic system, resulting in a short filtration cycle and very low cake moisture.

The term "tartrate" is used in this disclosure for indistinctly referring to the tartrate anion, the bitartrate anion and mixtures thereof. Relative concentration thereof in a determined liquid discharge or in a certain solution will depend on the equilibrium of the three coexisting species: tartaric acid-bitartrate-tartrate, taking into account their dissociation constants K₁=3.16 10⁻³ and K₂=6.3 10⁻⁵, and according to factors such as temperature, pH in the medium and the saline effect.

The first step of the method of the invention consists in putting in contact an anionic exchange resin (7) with said liquid discharge (1) from the wine-alcohol industry. Said liquid discharge can be stored in a suitable tank (2). Said resin (7) is characterized by interchanging chloride anions. A cationic solution (8) is obtained from the exchange, solution which can be stored in a suitable tank (3). Said cationic solution (8) comprises cations, the main one being potassium cation, chloride anions from the resin and non-ionic organic matter, whereas the resin (7) fixes anions present in the liquid discharge (1) such as tartrate, which is the one found in the greatest concentration, followed by the malate anion and other anions from the weak organic acids present in less proportion in the liquid discharge (1).

The cationic solution (8) obtained in the previous step can be concentrated without any salts precipitating in the form of chloride, until obtaining a potassium-enriched solution (10). The concentration takes place in a conventional evaporator (9) having a suitable evaporating capacity. Conventional evaporators used consist of a vacuum multiple effect system of long vertical tubes and top front feeding. In a particular embodiment the evaporator (9) used for concentrating the cationic solution (8) is of 6 effects, with a capacity of 20000 Kg/hour of evaporated water.

The cationic solution (8) can be concentrated with the only limitation of physico-chemical constants affecting solution fluidity, handling and storage (density and viscosity) in order to obtain a liquid fertilizer. The solution is normally concentrated so that the volume reduces between 30 to 20 parts to one, until reaching potassium purity values of commercial interest of about 3% potassium oxide, and with a significant content in trace elements. In a particular embodiment the cationic solution (8) is concentrated so that the volume reduces 25 parts to 1. Potassium-enriched solution (10) analysis shows the following composition, in which percentages are expressed in weight with respect to the total weight of the solution: organic matter 25-29%; potassium 3-3.5%; calcium 0.62%; magnesium 0.3%; iron 0.12%; sodium 0.1%; organic nitrogen 0.08%; inorganic nitrogen (N03 83 ppm; NH₃ 1.88 ppm); organic sulfur 0.3%; inorganic sulfur 0.01%; organic phosphorous 0.19%; inorganic phosphorous 80 ppm; copper 38 ppm; zinc 20 ppm; and pH 4.7. This potassium-enriched solution (10) corresponds, according to current legislation Real Decreto (Royal Decree) 824/2005 on fertilizing products (BOE (Official State Bulletin) No. 171 of July 19, 2005) to group 2, "Organic fertilizer", and more precisely to group 2.5, "NK liquid organic fertilizer of plant origin": a liquid product obtained in distillation of beetroot, cane or grape by-products. It is normally useful as a raw material for preparing other soluble plant mineral-organic fertilizers of Group 3.

The anionic exchange resin (7) used in the bed, for example, can be any strongly basic or medium basic anionic resin or mixtures thereof, characterized in that they exchange chloride anions. In a particular embodiment a 50% weight mixture is used of a strong resin, such as for example AMBERLITE FPA-97 Cl.-ROHM and HAAS and a medium basic anionic resin, such as for example LEWATIT MP-64 BAYER. The pH interval can be comprised between 0 and 8. The unitary operation of anionic exchange can be repeated cycle after cycle.

When the anionic exchange resin (7) is exhausted, it is regenerated using a sodium chloride solution (11) that is stored in a suitable tank (4). In a particular embodiment it has a 1.8 M concentration. In another particular embodiment it has a 1.43 M concentration. Chloride is fixed to the resin, and the retained anions elute together with the sodium cation coming from the sodium chloride solution. An anionic solution (12) is thus obtained comprising tartrate, malate and lactate anions, amongst others, corresponding to the so-called fixed acids of wine such as tartaric acid, malic acid, lactic acid and others, in their original proportions, together with sodium cations.

The method of the invention further comprises obtaining a tartaric acid-enriched solution (17) from the anionic solution (12) obtained in the aforementioned step d) of the method of the invention, comprising the steps of:
(Ai) putting into contact a cationic exchange resin (13) with the anionic solution (12) in order to obtain a solution (14) comprising tartaric acid; and
(Aii) concentrating said solution (14) in order to obtain the tartaric acid-enriched solution (17).

Alternatively, the method of the invention comprises obtaining a calcium tartrate precipitate (23), from said anionic solution (12) obtained in step d) described above of the method of the invention, comprising the steps of:
(Bi) treating the anionic solution (12) with calcium chloride (19) in order to obtain a calcium tartrate precipitate (20) that is kept in suspension; and
(Bii) recovering said precipitate and drying it in order to obtain calcium tartrate (C₄H₄O₆Ca.4H₂O) (23).

According to the first of the choices mentioned above, the cationic exchange resin (13) is put in contact with the anionic solution (12) retaining the sodium cations and releasing protons. A solution (14) is thus obtained from the resin (13) comprising organic acids tartaric, malic, lactic and others present in their original proportion in the liquid discharge (1). This solution (14), which can be stored in a tank (15) if so desired, is concentrated in a conventional evaporator (16) with a suitable evaporation capacity, in order to obtain a tartaric acid-enriched solution (17). The conventional evaporators used consist of a multiple effect, a vacuum multiple effect system of long vertical tubes and top front feeding. In a particular embodiment, the evaporator used for tartaric solution is a 2 effect evaporator with a capacity of 2000 Kg/hour of evaporated water.

The solution (14) can be concentrated with the only limitation of the physico-chemical constants that affect fluidity, handling and storage of the solution (density and viscosity). The tartaric acid-enriched solution (17) can be advantageously used as a natural wine acidity corrector for those wines that need it since it keeps the original proportion of acids present. This enological practice is usual, regulated and is usually performed only with industrial tartaric acid, which obviously tends towards disequilibrium between the acids present and thus affects wine equilibrium. However, the enriched solution (17) shows a deep-honey color, and when it is used for correcting pale white wine acidity it could affect their color intensity. Therefore, it is possible, if so required, to decolorize the starting liquid discharge (1) in order to avoid the deep-honey color of the enriched solution (17). For this, the liquid discharge (1) is decolorized before the filtration described above, with food-grade discoloring carbon with a low powder index such as ESSECO/EVERDEC W 98, usually used in wine and must discoloring. The use of absorbent resins leads to similar results and resins can be used such as AMBERLITE XAD 761-ROHM and HAAS or LEWATIT VP OC 1064 -BAYER.

The solution (14) is normally concentrated between 10 and 6 parts to one, usually obtaining solutions of variable concentration, normally comprised between 1.6 M and 1.33 M. In a particular embodiment the solution (14) is concentrated until it shows approximately 200 g/L of tartaric acid and approximately 68 g/L of malic acid. The total acidity measured using phenolphthalein as an indicator is of 4 equivalents/liter.

The unitary cationic exchange operation can be repeated cycle after cycle. The cationic exchange resin (13) in the bed, for example, can be any proton exchanging cationic resin, preferably a strong cationic resin, such as AMBERLITE FP-23 H ROHM and HAAS. The cationic exchange resin is regenerated using an effective amount of a hydrochloric acid solution (6) with a suitable concentration that is stored in a tank (5). Said concentration is normally comprised between 1.43 and 1.8 M HCl. 1.43 M hydrochloric acid is used in a particular embodiment. The pH interval at which this cationic exchange step can take place can be comprised between 0 and 14.

The method of the invention comprises, alternatively, obtaining a calcium tartrate precipitate (C₄H₄O₆Ca.4H₂O) (23), from the anionic solution (12) obtained from step d) mentioned above. According to this alternative, the anionic solution (12) is treated with calcium chloride (19) in a suitable tank (18) in order to initially obtain a calcium tartrate precipitate (20) in suspension. Said precipitate in suspension is recovered by conventional methods such as filtration or centrifugation in a suitable device (21), and subsequent drying in a suitable device (22) in order to obtain a calcium tartrate precipitate (C₄H₄O₆Ca.4H₂O) (23). A filtrate (11) is obtained from the precipitate recovery comprising sodium chloride, which can be piped and stored in a suitable tank (4). Said filtrate (11) can be reused in order to regenerate the anionic resin (7), which constitutes an additional advantage of the method of the invention.

The method of the invention can be carried out at different temperatures with the limitation set by the type of resin used. Thus, for example, anionic resins should not be used above temperatures exceeding 60°C. In a preferred embodiment the temperature is room temperature about 25°C.

In a particular embodiment the method of the invention is suitable for treating discharges from rectified must manufacture. Rectified must is a sweet liquid product originating from natural must that has been demineralized and discolored. Must is always a potassium bitartrate saturated solution the solubility product of which at 20°C is 7.07 10⁻⁴ and normally shows a C₄H₄O₆H⁻ concentration of about 0.0265 M and a K⁺ concentration of about 0.0265 M. The rectified must manufacturing system is analogous to that of complete water demineralization by means of ionic exchange resins and its manufacture comprises passing the natural grape must by four beds with strong cationic, weak anionic, strong anionic and strong cationic resin charges in the order described. The first resin retains all the cations, the second and third resins retain all the anions and the fourth has the function of bringing the pH to about 3.4, which corresponds to the pH value of the starting must. Once the resins are saturated they are regenerated; cationic resins are regenerated with a 1 N hydrochloric acid solution; and anionic resins with an alkaline 1 N sodium hydroxide solution. The acid solution contains all the cations (mainly potassium) in the presence of a single chloride anion, and the alkaline solution contains all the anions, mainly tartrate and malate in the presence of a single sodium cation. In a particular embodiment the acid solution has a 0.135 M concentration of potassium chloride and pH 2, and the alkaline solution has a 0.135 M concentration of sodium tartrate and pH 5. Currently the acid solutions are discharged to the environment. The alkaline solutions are usually also discharged as part of the detartarized lees vinasses or mother liquors.

However, these acid and alkaline solutions, of potassium and sodium tartrate respectively, are similar in composition and characteristics to the cationic solution (8) and the anionic solution (12) respectively and can be subjected to certain steps of the method of the invention. In this sense, the acid solution is incorporated as a cationic solution (8) to the method of the invention and is directly subjected to the evaporation step in the evaporator (9), obtaining from it a potassium-enriched solution (10). On the other hand, the alkaline sodium tartrate solution is incorporated as an anionic solution (12) to the method of the invention. Thus can be obtained from it either a tartaric acid-enriched solution (17) carrying out steps (Ai) and (Aii), or rather a calcium tartrate precipitate (23) carrying out steps (Bi) and (Bii) described above. The acid and alkaline solutions, of potassium and sodium tartrate respectively, are subjected to the corresponding steps of the method of the invention directly, without filtration since the grape must they come from is filtered prior to demineralization. In a particular embodiment, the acid solution has a concentration of 5 g/L of potassium cation and the sodium tartrate alkaline solution or anionic solution (12) has 20 g/L of tartrate anion and 6.5 g/L of malate anion.

One of the main advantages of the method of the invention lies in that the liquid discharges of the wine-alcohol industry are not only treated more efficiently than in the state of the art, but that at the same time it achieves their exploitation, obtaining value added products with commercial interest in the very wine-alcohol industry. The method of the invention allows recovering a liquid fertilizer with a high potassium content comprised between 3% and 3.5% together with other trace elements, and a tartaric acid-enriched solution and other weak acids present in the liquid discharges in the same proportion, which can be advantageously used in the very wine-alcohol industry, as an additive for correcting wine acidity or in calcium tartrate manufacture.

Another additional advantage lies in that after a first charge of sodium chloride to the system in which the method of the invention is being carried out, both if calcium chloride (19) and if a hydrochloric acid solution (6) is used, there is no sodium chloride (11) consumption, but rather it stays constant during the method. The sodium cation is constant in the system.

An additional advantage lies in that the water recovered in the conventional evaporators can be reused in a number of factory and winery services, marc washing, lees dilution, cooling towers, boilers, in some cases with prior pH conditioning to avoid corrosion attacks, and even for agricultural irrigation. The water may likewise be used in the method of the invention for preparing the sodium chloride, hydrochloric acid and/or calcium chloride solutions.

Examples of the method of the invention are exposed below for better comprehension of the invention and must in no case be considered a limitation of the scope thereof.

### EXAMPLES

### Example 1. Treatment of a wine vinasse

The method of the invention can be carried out in an apparatus such as that shown in Figure 1.

The liquid discharge (1) selected as starting material was a wine vinasse with a 0.026 M concentration of C₄H₄O₆HK. The volume to be processed was 400,000 liters.

The liquid discharge (1) was treated with an anionic exchange resin (7) consisting in a 50% mixture of the strong resin AMBERLITE FPA-97 Cl.- ROHM and HAAS and the medium resin LEWATIT MP-64 BAYER. The industrial installation has a load of 6,500 liters of strongly basic resin and 6500 liters of medium basic resin, with a total exchange capacity of 17,160 equivalents. The vinasse volume/liter of resin ratio is 7.78. 4 cycles of 100,000 liters each were performed. (Coefficient 7.69)

Initially 400,000 liters of cationic solution (8) were obtained with a 0.026 M concentration of potassium chloride. This solution was concentrated in an evaporator (9) until obtaining 16,300 liters of potassium-enriched solution (10) with a 0.638 M potassium chloride concentration, reducing the volume by 24.54 parts to 1. Said potassium-enriched solution (10) had a content of 3% by weight of potassium expressed as K₂O.

The evaporator (9) used for the potassium solution is of 6 effects with a capacity of 20,000 Kg/hour of evaporated water. The cationic solution (8) was fed at 20,000 liters/hour to produce 815 liters/hour of potassium-enriched solution (10) and 19,185 liters/hour of condensed water. The duration of the operation was of 20 hours.

The anionic exchange resin (7) was regenerated with 24,000 liters of sodium chloride solution (11) at a 1.43 M concentration, and 48,000 liters of anionic solution (12) were eluted with a tartrate concentration equivalent to 0.216 M tartaric acid.

The 48,000 liters of anionic solution (12) were treated with a strong cationic exchange resin (13) AMBERLITE FP-23 H ROHM and HAAS. The industrial installation carries a load of 7,695 liters with a total exchange capacity of 17,160 equivalents. The solution volume/liter of resin ratio is 7.78.

The solution (14) obtained comprising tartaric acid was concentrated in an evaporator (16) until obtaining 7,800 liters of tartaric acid-enriched solution (17) with a 1.33 M concentration in tartaric acid, the volume reducing in 6.15 parts to one, and with a tartaric acid content of 200 g/L. The evaporator used for the tartaric solution (16) was of 2 effects with a capacity of 2,000 Kg/hour of evaporated water. The anionic solution (14) was fed at 2,400 liters/hour in order to produce 390 liters/hour of tartaric acid-enriched solution (17) and 2,010 liters/hour of condensed water. Operation duration was 20 hours.

The cationic exchange resin (13) was regenerated with 48,000 liters of 1.43 M hydrochloric acid solution (6).

## Claims

1. A method for treating a liquid discharge originating from the wine-alcohol industry comprising the following steps:
a) Putting an anionic exchange resin in contact with a liquid discharge from the wine-alcohol industry in order to obtain a cationic solution comprising potassium cations and chloride anions;
b) Concentrating said cationic solution obtained in the previous step in order to obtain a potassium-enriched solution;
c) Regenerating the anionic exchange resin used in step a) with a sodium chloride solution; and
d) obtaining an anionic solution comprising tartrate anions and sodium cations.

2. A method according to claim 1, further comprising obtaining a tartaric acid-enriched solution from the anionic solution obtained in the previous step d) comprising the steps of:
(Ai) putting in contact a cationic exchange resin with said anionic solution in order to obtain a solution comprising tartaric acid; and
(Aii) concentrating said solution obtained in step (Ai) in order to obtain the tartaric acid-enriched solution.

3. A method according to claim 1, further comprising obtaining a calcium tartrate precipitate from the anionic solution obtained in previous step d) comprising the steps of:
(Bi) treating said anionic solution with calcium chloride in order to obtain a calcium tartrate precipitate that is kept in suspension; and
(Bii) recovering said precipitate and drying it in order to obtain calcium tartrate.

4. A method according to claim 1, **characterized in that** the liquid discharge is a liquid discharge selected from a pomace vinasse, a wine vinasse, a lees vinasse, winery tank wash water, machine wash water and mixtures thereof.

5. A method according to claim 1, wherein the anionic exchange resin is **characterized in that** it exchanges chloride anion and is selected from a strong basic resin, a medium basic resin and mixtures thereof.

6. A method according to claim 2, wherein the cationic exchange resin is **characterized in that** it exchanges protons.

7. A method according to either of claims 1 or 2, **characterized in that** the concentration of the cationic solution and of the solution comprising tartaric acid is performed in an evaporator.

8. A method according to claim 1, **characterized in that** the cationic solution is concentrated between 30 and 20 parts to one.

9. A method according to claim 2, **characterized in that** the solution comprising tartaric acid is concentrated between 10 and 6 parts to one.

10. A method according to claim 1, **characterized in that** the potassium-enriched solution comprises between 25% and 29% by weight with respect to the weight of the total solution of organic matter and between 3% and 3.5% by weight with respect to the weight of the total solution of potassium.

11. A method according to claim 2, **characterized in that** the tartaric acid-enriched solution comprises approximately 200 grams/liter of tartaric acid and approximately 68 grams/liter of malic acid.

12. A potassium-enriched solution obtained by the method of claim 1.

13. A tartaric acid-enriched solution obtained by the method of claim 2.

14. A calcium tartrate precipitate obtained by the method of claim 3.
